# EUROPEAN PATENT SPECIFICATION

(11) **EP 1 390 025 B1**
(45) Date of publication and mention of the grant of the patent: **30.03.2005**
(21) Application number: 02724475.5
(22) Date of filing: 09.05.2002
(51) Int. Cl.: A61K 31/202, A23L 1/30

(54) **USE OF COENZYME Q (UBIQUINONE) AND EICOSAPENTAENOIC ACID (EPA) FOR THE TREATMENT OF NON-HODGKIN'S LYMPHOMA AND PSYCHIATRIC OR NEUROLOGICAL DISORDERS**
VERWENDUNG VON COENZYM Q (UBIQUINONE) UND EIKOSAPENTAENSÄURE (EPA) FÜR DIE BEHANDLUNG VON NON-HODGKIN'S LYMPHOMA UND PSYCHIATRISCHEN ODER NEUROLOGISCHEN ERKRANKUNGEN
UTILISATION DU COENZYME Q (UBIQUINONE) ET DE L' ACIDE EICOSAPENTAENOIQUE (EPA) POUR LE TRAITEMENT DES LYMPHOMES HODGKINIENS ET DES AFFECTIONS PSYCHIATRIQUES OU NEUROLOGIQUES

(30) Priority: 30.05.2001 GB 0113104; 28.09.2001 GB 0123446
(43) Date of publication of application: 25.02.2004
(73) Proprietor: Laxdale Limited, Stirling FK7 9JQ (GB)
(72) Inventor: HORROBIN, David Frederick, Kings Park House, Stirling FK7 8JQ (GB)
(74) Representative: Wakerley, Helen Rachael
(86) International application number: PCT/GB2002/002148
(87) International publication number: WO 2002/096408

(56) References cited:
- WO-A-00/23069
- CA-A- 2 052 577
- GB-A- 2 229 363
- JIALAI I ET AL.: "correspondence gissi-prevenzione trial" THE LANCET, vol. 354, 30 October 1999 (1999-10-30), page 1556 XP001094504
- PALAKURTHI S S ET AL: "Inhibition of translation initiation mediates the anticancer effect of the n-3 polyunsaturated fatty acid eicosapentaenoic acid." CANCER RESEARCH. UNITED STATES 1 JUN 2000, vol. 60, no. 11, 1 June 2000 (2000-06-01), pages 2919-2925, XP001093871 ISSN: 0008-5472
- KARLSSON J: "Plasma Omega-3 Fatty Acids Before and After Nutritional Therapy" JOURNAL OF NUTRITIONAL AND ENVIRONMENTAL MEDICINE, vol. 8, no. 1, 1998, pages 25-34, XP000892752

## Description

Eicosapentaenoic acid (EPA, 20:5n-3) is a highly unsaturated fatty acid which has therapeutic uses in a number of illnesses. For example, it has value in cardiovascular disease because it lowers triglyceride levels, produces vasodilatation and has cardiac anti-arrhythmic effects. It has anti-inflammatory effects in illnesses such as rheumatoid arthritis, osteoarthritis and inflammatory bowel disease. It has beneficial effects in psychiatric and neurological disorders such as schizophrenia, depression and Huntington's disease. It has anti-cancer effects particularly in the relief of cancer cachexia.

Usually EPA is exceptionally safe and has no important side effects other than mild gastrointestinal disorders. The Food and Drug Administration (FDA) of the USA has determined that, in doses of up to 3g/day when present in fish oils, EPA is Generally Recognised as Safe (GRAS) for the general population.

Although eicosapentaenoic acid is usually administered in the form of fish or other marine oils or in the form of mixed ethyl esters where it is mixed with many other fatty acids, including docosahexaenoic acid (DHA), it has recently been found by the applicant and their collaborators that its therapeutic effects are considerably enhanced by making purer and purer preparations of eicosapentaenoic acid or its derivatives. Particularly effective preparations are those where docosahexaenoic acid or its derivatives are present at low or very low levels or are absent (WO 00/44361). Particularly valuable are preparations where the eicosapentaenoic acid level is greater than 90% or preferably greater than 95% and where the docosahexaenoic acid contact is below 5%, and preferably below 1%. Linoleic acid may interfere with the action of eicosapentaenoic acid and so it is also valuable if the linoleic acid content of the formulation is below 5% and preferably below 1%.

The present invention addresses a hitherto unknown possible side effect of EPA when used in high doses and in purified forms and provides a way of alleviating this side effect. The invention finds application not only with EPA, but also with other therapeutically significant essential fatty acids, particularly the eicosapentaenoic acid precursor, stearidonic acid (18:4 n-3) and the omega-6 fatty acids gamma-linolenic acid (18:3 n-6), dihomogamma-linolenic acid (20:3 n-6) and arachidonic acid (20:4 n-6).

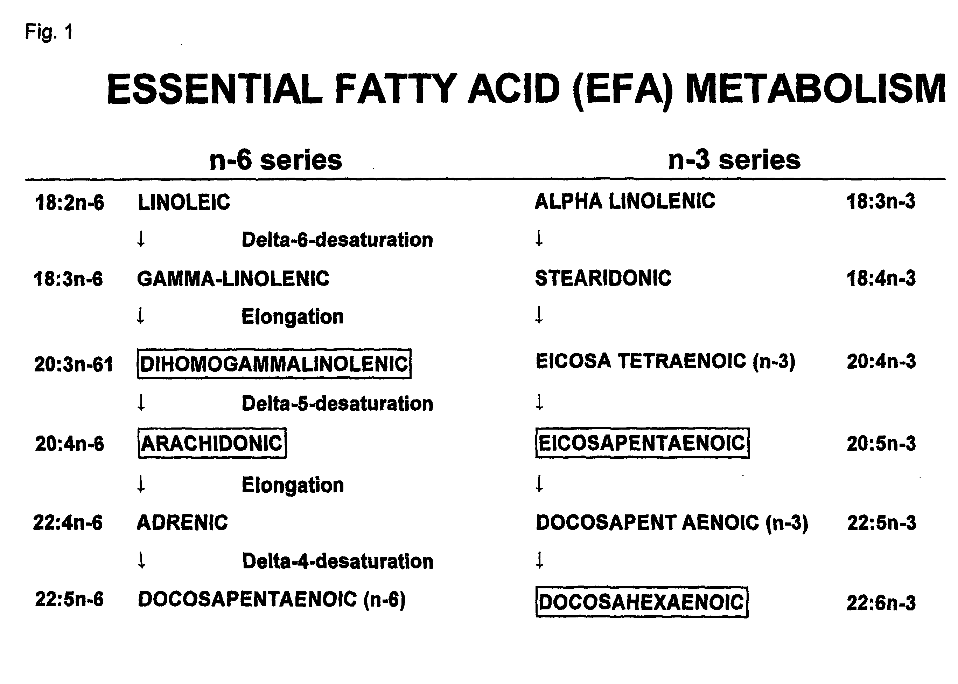

The pathways of metabolism of the unsaturated essential fatty acids (EFAs) are shown in figure 1. The EFAs are like vitamins in the sense that they are required for human and animal metabolism but cannot be synthesised de novo by the mammalian body. There are two sorts of EFAs: the n-6 (or omega-6) and the n-3 (or omega-3). The parent compounds linoleic acid (LA) of the n-6 series and alpha-linolenic acid (ALA) of the n-3 series are the main compounds found in the diet. However, to be useful to the body, these parent compounds must be converted to the so-called derived essential fatty acids shown in figure 1. These derived EFAs play key roles in the structures of all internal and external cell membranes. They are also released from these cell membranes following many different types of cell activation which convert phospholipases A₂, C and D to active forms and which directly or indirectly lead to release of the free acids from membrane phospholipids.

The present invention provides the co-administration of ubiquinone (coenzyme Q) with EPA. This may most conveniently be done by incorporating the ubiquinone in the formulation with the EPA. The present invention provides formulations for pharmaceutical or nutritional use which contain eicosapentaenoic acid (EPA) and ubiquinone in any appropriate assimilable form.

These formulations would specifically not contain any form of ascorbic acid or ascorbate, which the inventors have found to make the eicosapentaenoic acid formulation unstable. Further, the present formulations do not contain any other triglyceride lowering drug, such as pantethine, because the triglyceride lowering effect of the drug might be inappropriately large while using purified eicosapentaenoic acid. The unsaturated acid itself has a triglyceride lowering effect. Further, the invention is not directed to the formulations in which the fatty acid preparation is combined in the same dosage form or same pack with an enzyme inhibitor selected from an inhibitor of COX-1 and/or COX-2, an inhibitor of LOX and an inhibitor of one or more of the FACL enzymes.

The present invention also provides the use of ubiquinone (coenzyme Q) and EPA in the manufacture of a medicament for treatment of patients suffering from any disorder conventionally treated with EPA, as well as a method of treatment or prevention of such disorders by the co-administration of ubiquinone (coenzyme Q) and EPA. Such disorders include cardiovascular and cerebrovascular diseases; diseases where inflammation plays a role, including rheumatoid arthritis and osteoarthritis, inflammatory bowel disorders, endometriosis and asthma; disorders of abnormal and painful muscle contractions, including irritable bowel or bladder syndromes, dysmenorrhoea or skeletal muscle spasms; disorders of the functioning of the central and peripheral nervous systems, including psychiatric and neurological disorders; cancer and cancer-associated syndromes, including cachexia.

Preferably, the ratio of EPA to ubiquinone is between 2000:1 and 1:1, and still more preferably between 200:1 and 3:1.

The EPA may be in the form of the free acid, a sodium, potassium, lithium or other salt, any ester, including an ethyl ester or a cholesterol ester, an amide, a phospholipid, or a tri-, di- or monoglyceride. Other derivatives which are able to raise the levels of the fatty acid in the blood or tissues may be used. The preferred form of EPA is the ethyl ester or the triglyceride. These are particularly well tolerated by the gastrointestinal tract.

The eicosapentaenoic acid or derivative should have a purity level such that interference at the key points of biological action from other fatty acids or fatty acid derivatives is reduced. The eicosapentaenoic acid or derivative present in the eicosapentaenoic acid preparation of the present formulations should be at least 70% pure and preferably at least 80% pure. It is especially preferred that the eicosapentaenoic acid or derivative is 90% or 95% pure. In particular, there are two essential fatty acids which are commonly found in oils and which can play a role in the interference of the actions of the therapeutic fatty acids: docosahexaenoic acid and linoleic acid. It is a requirement that the docosahexaenoic acid or derivative and the linoleic acid or derivative present is each less than 10%, preferably less than 5% and very preferably less than 1% of any preparation of a fatty acid or fatty acid derivative used in the formulations of the present invention.

It is advantageous that the daily dose of EPA is between 100mg and 100g and the daily dose of ubiquinone is between 10mg and 2000mg. Preferably, the daily dose of EPA is between 0.5g and 20g and the daily dose of ubiquinone is between 50mg and 500mg.

The formulations of the invention are preferably formulated for oral administration or enteral administration. They may instead be formulated for topical, vaginal or rectal administration, or for parenteral administration via intravenous, intramuscular or subcutaneous routes.

Although this co-administration is likely to be of particular value when using very high doses of EPA, it will also be useful at any dose of EPA to reduce the risk of any adverse effect and also to help to protect the EPA against the risk of *in vivo* oxidation.

Ubiquinone, also known as coenzyme Q, is a molecule which is found in most cells in the human body (L Ernster & G Dallner, Biochimica Biophysica Acta 1995; 1271: 195-204). Its structure is shown:

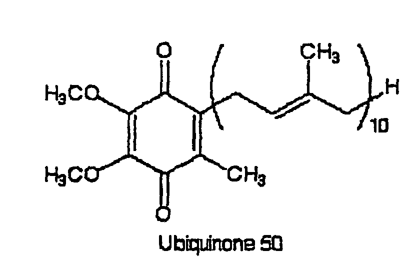

It consists of a quinone ring with a polprenylated side chain. The number of isoprene units in the side chain is variable but each species has a typical number which is 10 in humans. Ubiquinone has two main identified roles in the body. First it is a component of the respiratory chain in mitochondria where it is required for the normal transport of electrons and hence the normal generation of energy. Second it has potent anti-oxidant properties and so may help to prevent the generation of potentially harmful free radicals from the oxidation of highly unsaturated fatty acids such as EPA.

Other highly unsaturated fatty acids may also be used in therapy and may give rise to free radicals which might theoretically give rise to adverse effects. Such fatty acids include in particular the omega-6 fatty acids gamma-linolenic acid, dihomogammalinolenic acid and arachidonic acid, as well as the eicosapentaenoic acid precursor, stearidonic acid.

In a further aspect of the invention, there are provided formulations of these fatty acids or derivatives which contain coenzyme Q, as well as methods of treatment or prevention of disorders involving the co-administration of coenzyme Q with any of these fatty acids or derivatives mentioned above. The EPA in any of the above formulations of the present invention may be supplemented with or replaced by one or more highly unsaturated fatty acids drawn from the group gamma-linolenic acid, dihomogammalinolenic acid, arachidonic acid and stearidonic acid. As with EPA, the fatty acid preparations used for making the formulations of the present invention should contain more than 70% of the particular fatty acid, preferably more than 80% or 90%, and very preferably more than 95%. They should also contain less than 10%, preferably less than 5% and very preferably less than 1% of docosahexaenoic acid or of linoleic acid.

There may be circumstances in which it is appropriate to combine one of these preparations containing 70% or more of one fatty acid with one of more other preparations containing 70% or more of another fatty acid. For example, in situations where it is desirable to combine a fatty acid of the n-3 series with one of the n-6 series, an EPA and/or SA preparation may be combined with an GLA and/or a DGLA or an AA preparation to make the final product.

The EPA and the other fatty acids may be provided in any assimilable form which leads to a rise in the concentration of EPA or other fatty acid in either plasma or red cell membranes or in tissues to which the formulation is directly applied, such as the skin. Such a change in EPA or other fatty acid concentration can readily be determined by standard techniques such as gas chromatography following separation of the lipid fraction by standard lipid extraction. Lipid extraction may optionally be followed by separation of lipid classes by thin layer chromatography or high pressure liquid chromatography prior to gas chromatography. The EPA or other fatty acid may be provided as the free fatty acid, as a salt, as an ester such as the ethyl ester, as an amide, as a phospholipid, as a tri-, di- or monoglyceride or in any other assimilable form.

The daily dose of EPA or other fatty acid may be from 100mg to 100g, but is preferably within the range of 0.5g to 20g. The concentration of EPA or other fatty acid in the EPA-containing material provided for the formulation may be anything above 70% but is preferably above 80% or 90% and very preferably above 95%.

The daily dose of ubiquinone provided in the formulation may be from 10mg to 2000mg per day but is preferably in the range of 50mg to 500mg per day. The ratio of EPA or other fatty acid to ubiquinone may be from 2000:1 to 1:1 but is preferably in the range of 200:1 to 3:1.

The EPA/other fatty acid preparation and ubiquinone may be incorporated into any appropriate formulation or vehicle for either oral administration, enteral administration, parenteral administration by intravenous, intramuscular or subcutaneous routes, or for topical administration via the skin or rectal or vaginal administration by suppositories or pessaries.

Work has shown the administration of essential fatty acids (EFAs), including eicosapentaenoic acid, to have therapeutically beneficial results in the treatment of many diseases. The advantages of the present invention will be therefore widespread. Case studies follow, but the applications are expected to be diverse, based on the present and future knowledge of the uses of EFAs. Examples of granted patents which demonstrate that these fatty acids have a wide range of utility in many diseases are the following US cases: US 4,826,877; 5,847,000; 5,457,130; 4,302,447; 4,681,896; 5,198,468; 5,922,345.

The formulations of the present invention are suited for the treatment of any form of cancer and cancer cachexia and the present invention further provides such treatment and the use of the combination of the fatty acid preparation with ubiquinone in a method of manufacture of a medicament for the treatment of cancer or cancer cachexia.

The present invention further provides the treatment or control of abnormal cardiac rate and rhythm and the use of the combination of the fatty acid preparation with ubiquinone in a method of manufacture of a medicament for the treatment or control of abnormal cardiac rate and rhythm.

The formulations are also suited for the treatment of any form of psychiatric disease including schizophrenia, schizoaffective disorders, schizotypy, depression, anxiety, bipolar disorder, mania, borderline personality disorder, alcoholism and attention deficit hyperactivity disorder or any other psychiatric illness and the present invention provides such treatment and the use of the combination of the fatty acid preparation with ubiquinone in a method of manufacture of a medicament for the treatment of any such psychiatric disease.

The formulations may be used in the treatment of any form of neurological or neurodegenerative disease including Parkinson's disease, Alzheimer's disease, amyotrophic lateral sclerosis, Huntington's disease and other "triplet-repeat" diseases, stroke, multi-infarct and other forms of dementia, multiple sclerosis, chronic fatigue and epilepsy and the present invention provides such treatment and the use of the combination of the fatty acid preparation with ubiquinone in a method of manufacture of a medicament for the treatment of any such neurological or neurodegenerative disease.

The formulations are suited for the treatment of any form of inflammatory disease including any form of arthritis, any form of inflammatory skin disease including psoriasis and eczema, asthma, any form of inflammatory gastrointestinal disease including ulcerative colitis and Crohn's disease, and any inflammatory conditions of any other organs including the kidneys, the reproductive system, the eyes and the brain and the present invention provides such treatment and the use of the combination of the fatty acid preparation with ubiquinone in a method of manufacture of a medicament for the treatment of any such inflammatory disease.

The formulations may be used in the treatment of any form of cardiovascular or cerebrovascular disease and the present invention provides such treatment and the use of the combination of the fatty acid preparation with ubiquinone in a method of manufacture of a medicament for the treatment of any cardiovascular or cerebrovascular disease.

The formulations may be used in the treatment of any form of respiratory disease, including asthma or chronic obstructive pulmonary disease, and the present invention provides such treatment and the use of the combination of the fatty acid preparation with ubiquinone in a method of manufacture of a medicament for the treatment of any respiratory disease, including asthma or chronic obstructive pulmonary disease.

The formulations may be used in the treatment of any form of metabolic disease including diabetes, syndrome X, and any disturbance of calcium metabolism including osteoporosis, urolithiasis, or urinary tract stone formation and the present invention provides such treatment and the use of the combination of the fatty acid preparation with ubiquinone in a method of manufacture of a medicament for the treatment of any such metabolic disease.

The formulations may be used in the treatment of any form of renal or urinary tract disease and the present invention provides such treatment and the use of the combination of the fatty acid preparation with ubiquinone in a method of manufacture of a medicament for the treatment of any renal or urinary tract disease.

The formulations may be used in the treatment of any form of disease or disorder of the reproductive system or menstrual cycle, including breast pain, premenstrual syndrome, dysmenorrherea or endometriosis, and the present invention provides such treatment and the use of the combination of the fatty acid preparation with ubiquinone in a method of manufacture of a medicament for the treatment of disease or disorder of the reproductive system or menstrual cycle, including breast pain, premenstrual syndrome, dysmenorrherea or endometriosis.

The formulations may be used in the treatment of any form of disease or disorder characterised by abnormal and painful muscle contractions, including irritable bowel or bladder syndromes or skeletal muscle spasms, and the present invention provides such treatment and the use of the combination of the fatty acid preparation with ubiquinone in a method of manufacture of a medicament for the treatment of disease or disorder characterised by abnormal and painful muscle contractions, including irritable bowel or bladder syndromes or skeletal muscle spasms.

Eicosapentaenoic acid has been suggested to be a fatty acid with anti-cancer potential. SS Palakurthi et al (Cancer Research 2000; 60: 2919-25) reported that EPA could inhibit the growth of cancerous cells and reported previous literature describing similar findings. However neither Palakurthi et al, nor the previous literature have provided any information as to how these laboratory findings might be translated into the treatment of human cancer. A granted European patent (EPO 464084) describes the use of EPA in the treatment of weight loss associated with cancer but does not discuss the possibility that EPA might be used to treat the underlying cancer itself.

The following example reports that cancer developed in an individual who had been taking ethyl-EPA at a regular dose of 1g/d. This individual had also at times in the past three years taken 2g/d EPA but still cancer developed. It can therefore be concluded that doses of EPA which are 2g/day or less are likely to be ineffective in the management of cancer. However, when the dose of EPA was increased to 10g/day, within a matter of days the cancer reduced dramatically in size. This shows therefore that a dose of EPA itself of 10g/d does have a direct anticancer effect. Doses of 3g/d to 30g/d, and preferably of 5g/d or 20g/d will therefore be useful in the treatment of cancer. The EPA may be provided in any assimilable form. However, the triglyceride form and the ethyl-ester form are relatively non-irritant to the gastrointestinal tract and are the preferred forms for delivering these large amounts by mouth although other forms could be used for the intravenous route of administration. The EPA for oral administration should be in the purest form possible, at least 70% pure, preferably 80 - 90% pure, and very preferably 95% or more pure. Docosahexaenoic acid and linoleic acid should each be present at less than 5% and preferably less than 1%. The EPA may be formulated in capsules, liquids or emulsions such as may be required to make it appropriate for either oral or intravenous administration.

The example also describes the development of cardiac arrhythmias when high doses of EPA were administered, arrhythmias which were successfully controlled by the co-administration of coenzyme Q. However such arrhythmias may not always develop when high doses of EPA are administered and so we claim the administration of EPA itself in high doses of 3g/d or more for the treatment of cancer.

### Examples

A 61 year old male who had been taking 1g/d of ethyl eicosapentaenoate as a supplement for general health reasons developed a form of non-Hodgkin's lymphoma called a mantle cell lymphoma. This is characterised by an over-production of a cell cycle controlling factor known as cyclin D1. Cyclin D1 appears to force the cells to divide continually. It has been reported that *in vitro* and in animal models of cancer, EPA is able to inhibit the synthesis of cyclin D1 (SS Palakurthi et al, Cancer Research 2000; 60: 2919-25). However, high doses are required to achieve the necessary levels in the cancer tissue.

It was therefore decided to give the patient 10g/d of 96% pure ethyl eicosapentaenoate containing less than 1% docosahexaenoic acid and no detectable linoleic acid. On the sixth day of treatment the patient's palpable tumours began to regress suggesting that the EPA was having a beneficial effect. The patient noticed no important side effects from EPA therapy. However, on the tenth day his heart rate became markedly irregular in both rate and rhythm: it oscillated from a low of around 60/min to a high of around 150/min and exhibited great irregularity.

No certain cause of the abnormal rhythm was identified. One possibility may have been the high intake of EPA. EPA at very high doses might theoretically give rise to oxygenated metabolites which could disturb cardiac rhythm. EPA at low doses seems to be strongly anti-arrhythmic but it is known that many anti-arrhythmic drugs can have pro-arrhythmic actions at certain doses.

Ubiquinone may have favourable effects on cardiac rhythm (RB Singh et al, Cardiovascular Drugs and Therapy 1998; 12: 347-353). 120mg/day was given to the patient taking high dose EPA. On the second day the heart rate and rhythm returned to normal, the rhythm being even and the rate at rest oscillating in the range of 65-75/min. The ubiquinone was then stopped after ten days. Four days after that the variability of rate and rhythm returned. On reintroducing the ubiquinone, the normal rate and rhythm came back. The patient therefore continued to take both the ubiquinone and high dose of EPA with no return of the cardiac rate and rhythm abnormality over a period of four months.

### Example Formulations

1. Soft or hard gelatin capsules containing 500mg of EPA in ethyl ester form where the purity of the ethyl-EPA is over 90% and where the docosahexaenoic acid and linoleic acid are each below 5% and where 30mg of ubiquinone is added. Between one and 30 such capsules may be taken daily.
2. Liquid 95% pure ethyl-EPA, where the DHA and linoleic acid are each less than 1%, flavoured to make it palatable, containing 50mg of ubiquinone per g. To be taken orally in doses from 0.5g to 20g/day.
3. Micro-encapsulated 80% pure ethyl-EPA, where the DHA is less than 10%, containing 100mg of ubiquinone/g. To be taken in doses providing between 100mg and 20g of EPA per day.
4. As examples 1-3 but where the EPA is provided in free acid, sodium salt, lithium salt, amide, phospholipid, mono- di- or triglyceride, or any other assimilable form.
5. As examples 1-4 but where the EPA is provided in a form which contains more than 70% of eicosapentaenoic acid and where the DHA and linoleic acid contents are each less than 10%.
6. As examples 1-5 but where the EPA and ubiquinone are provided in a formulation suitable for parenteral administration by intravenous, intramuscular or subcutaneous routes.
7. As examples 1-5 but where the EPA and ubiquinone are provided in a formulation suitable for topical administration.
8. As examples 1-7 but where the EPA is replaced by or supplemented with one or more fatty acids selected from gamma-linolenic acid, dihomogammalinolenic acid, arachidonic acid and stearidonic acid.

## Claims

1. Use in the manufacture of a medicament for the treatment of any of the following diseases or disorders:
non-Hodgkin's lymphoma;
schizophrenia, schizoaffective disorders, schizotypy, depression, anxiety, bipolar disorder, mania, borderline personality disorder, alcoholism and attention deficit hyperactivity disorder or any other psychiatric illness;
Parkinson's disease, Alzheimer's disease, Huntington's disease or any other "triplet repeat" disease, amyotrophic lateral sclerosis, dementia, multi-infarct dementia, multiple sclerosis, chronic fatigue and epilepsy;
of an eicosapentaenoic acid (EPA) preparation containing more than 90% EPA and less than 5% docosahexaenoic acid (DHA)and less than 5% linoleic acid; and
ubiquinone in any appropriate assimilable form.

2. Use according to claim 1, in which the EPA preparation contains more than 95% EPA and less than 1% docosahexaenoic acid (DHA)and less than 1% linoleic acid.

3. Use according to claim 1, in which the ratio of EPA to ubiquinone is between 2000:1 and 1:1.

4. Use according to claim 3, in which the ratio of EPA to ubiquinone is between 200:1 and 3:1.

5. Use according to claim 1, in which the EPA is in the form of the free acid, a sodium, lithium or other salt, any ester, an amide, a phospholipid, and a tri- di- or monoglyceride.

6. Use according to claim 5, in which the EPA is in the form of the ethyl ester.

7. Use according to claim 1, in which the medicament comprises a daily dose of EPA of between 100mg and 100g and a daily dose of ubiquinone is between 10mg and 2000mg.

8. Use according to claim 7, in which the daily dose of EPA is between 0.5g and 20g and the daily dose of ubiquinone is between 50mg and 500mg.

## Patentansprüche

1. Verwendung zur Herstellung eines Medikamentes zur Behandlung einer der folgenden Krankheiten oder Störungen:
Non-Hodgkin-Lymphom;
Schizophrenie, schizoaffektive Störungen, Schizotypie, Depressionen, Angst, bipolare Störungen, Manie, Borderline-Persönlichkeitsstörung, Alkoholismus und Aufmerksamkeitsdefizit-Hyperaktivitätsstörung oder eine beliebige andere psychiatrische Krankheit;
Parkinsonsche Krankheit, Alzheimersche Krankheit, Huntingtonsche Krankheit oder eine beliebige andere "Triplet-Repeat"-Krankheit, amyotrophe Lateralsklerose, Demenz, Multiinfarkt-Demenz, multiple Sklerose, chronische Müdigkeit und Epilepsie;
eines Eicosapentaensäure-(EPA)-Präparates mit mehr als 90 % EPA und weniger als 5 % Docosahexaensäure (DHA) und weniger als 5 % Linolsäure; und
von Ubichinon in einer beliebigen angemessenen assimilierbaren Form.

2. Verwendung nach Anspruch 1, wobei das EPA-Präparat mehr als 95 % EPA und weniger als 1 % Docosahexaensäure (DHA) und weniger als 1 % Linolsäure enthält.

3. Verwendung nach Anspruch 1, wobei das Verhältnis zwischen EPA und Ubichinon zwischen 2000:1 und 1:1 liegt.

4. Verwendung nach Anspruch 3, wobei das Verhältnis zwischen EPA und Ubichinon zwischen 200:1 und 3:1 liegt.

5. Verwendung nach Anspruch 1, wobei die EPA in Form der freien Säure, eines Natrium-, Lithium- oder anderen Salzes, eines beliebigen Esters, eines Amids, eines Phospholipids und eines Tri-, Di- oder Monoglycerids vorliegt.

6. Verwendung nach Anspruch 5, wobei die EPA in Form des Ethylesters vorliegt.

7. Verwendung nach Anspruch 1, wobei das Medikament eine EPA-Tagesdosis zwischen 100 mg und 100 g und eine Ubichinon-Tagesdosis zwischen 10 mg und 2000 mg umfasst.

8. Verwendung nach Anspruch 7, wobei die EPA-Tagesdosis zwischen 0,5 g und 20 g liegt und die Tagesdosis von Ubichinon zwischen 50 mg und 500 mg liegt.

## Revendications

1. Utilisation dans la fabrication d'un médicament pour le traitement de l'une quelconque des maladies ou troubles suivants:
lymphome non de Hodgkin;
schizophrénie, troubles schizo-affectifs, schizotypie, dépression, anxiété, désordre maniaco-dépressif, manie, trouble de la personnalité limite, alcoolisme et trouble de déficit de l'attention/hyperactivité ou toute autre maladie psychiatrique;
maladie de Parkinson, maladie d'Alzheimer, maladie de Huntington ou toute autre maladie à triplets répétés, sclérose latérale amyotrophique, démence par infarctus multiples, sclérose en plaques, fatigue chronique et épilepsie;
d'une préparation à base d'acide éicosapentaénoïque (EPA) contenant plus de 90% d'EPA et moins de 5% d'acide docosahexaénoïque (DHA) et moins de 5% d'acide linoléique; et
de l'ubiquinone sous toute forme assimilable appropriée.

2. Utilisation selon la revendication 1, dans laquelle la préparation à base d'EPA contient plus de 95% d'EPA et moins de 1% d'acide docosahexaénoïque (DHA) et moins de 1% d'acide linoléique.

3. Utilisation selon la revendication 1, dans laquelle le rapport d'EPA à ubiquinone est d'entre 2000:1 et 1:1.

4. Utilisation selon la revendication 3, dans laquelle le rapport d'EPA à ubiquinone est d'entre 200:1 et 3:1.

5. Utilisation selon la revendication 1, dans laquelle l'EPA est sous la forme de l'acide libre, d'un sodium, d'un lithium ou d'autre sel, d'un ester quelconque, d'un amide, d'un phospholipide et d'un tri-, di- ou monoglycéride.

6. Utilisation selon la revendication 5, dans laquelle l'EPA est sous la forme de l'ester éthylique.

7. Utilisation selon la revendication 1, dans laquelle le médicament comprend une dose quotidienne d'EPA d'entre 100 mg et 100 g et une dose quotidienne d'ubiquinone est d'entre 10 mg et 2000 mg.

8. Utilisation selon la revendication 7, dans laquelle la dose quotidienne d'EPA est d'entre 0,5 g et 20 g et la dose quotidienne d'ubiquinone est d'entre 50 mg et 500 mg.
